# EUROPÄISCHE PATENTSCHRIFT

(11) **EP 3 067 010 B1**
(45) Veröffentlichungstag und Bekanntmachung des Hinweises auf die Patenterteilung: **31.05.2017**
(21) Anmeldenummer: 15000674.0
(22) Anmeldetag: 07.03.2015
(51) Int. Cl.: A61C 9/00, A61B 5/00

(54) **Halterung für einen Intraoralscanner**
Holder for an intra-oral scanner
Support pour un scanner intra-oral

(43) Veröffentlichungstag der Anmeldung: 14.09.2016
(73) Patentinhaber: a.tron3d GmbH, 9020 Klagenfurt am Wörthersee (AT)
(72) Erfinder: Jesenko, Jürgen, 9584 Finkenstein (AT); Isola, Franz, 9545 Radenthein (AT)
(74) Vertreter: Beer & Partner Patentanwälte KG

(56) Entgegenhaltungen:
- EP-A2- 2 774 576
- DE-A1-102007 013 355
- DE-U1- 29 618 387
- US-A- 3 884 222

## Beschreibung

Die Erfindung betrifft eine Halterung zur Verwendung in Verbindung mit einem Handstück eines Intraoralscanners, welches einen Kopfbereich aufweist, und mit einem Grundkörper mit einem Aufnahmebereich, in welchem der Kopfbereich aufnehmbar ist.

Grundsätzlich lassen sich derartige Halterungen in zwei Arten unterteilen: Zum einen Halterungen, die das Handstück des Intraoralscanners an einem Griffbereich des Handstückes lagern und zum anderen Halterungen, die das Handstück an einem Kopfbereich des Handstücks lagern. Letztere sind beispielsweise aus der AT 13546 U1 bekannt.

Halterungen, die das Handstück am Griffbereich lagern, haben den Vorteil, dass das Kopfstück, welches in die Mundhöhle eines Patienten gebracht wird, nur mit der Mundhöhle und den darin befindlichen Objekten in Berührung kommt. So können Verschmutzungen, wie beispielsweise Speichel oder Blut, welche bei der Verwendung des Intraoralscanners an den Scannerkopf kommen können, nicht auf andere Oberflächen übertragen werden. Diese Halterungen sind daher sehr hygienisch. Allerdings haben sie den großen Nachteil, dass sie für eine Bedienungsperson sehr unkomfortabel zu verwenden sind, da die Halterung beim Ergreifen des Griffbereichs im Weg ist. Halterungen, die das Handstück des Scanners im Griffbereich lagern/halten, sind daher nicht wünschenswert.

Es gibt Lösungsansätze, Halterungen, die das Handstück im Griffbereich halten, zu verkleinern und so Platz für eine leichtere Handhabung zu schaffen. Allerdings sind diese nicht sehr stabil und sobald die Halterung (gegebenenfalls gemeinsam mit dem Scanner oder auch an einem Arm eines Behandlungsstuhles) bewegt wird, besteht die Gefahr, dass sich das Handstück aus der Halterung löst. Ein Herunterfallen des Handstücks ist dabei sowohl aus technischer Sicht unerwünscht, da im Handstück befindliche elektronische und/oder optische Elemente beschädigt werden können, als auch aus hygienischer Sicht, da das Handstück bei einem unbeabsichtigten Lösen aus der Halterung mit unsauberen Oberflächen wie dem Fußboden in Berührung kommen oder seinerseits Oberflächen verschmutzen kann.

Eine im Vergleich zu den vorgenannten Ansätzen wesentlich komfortablere Lösung bieten Halterungen, welche das Handstück im Wesentlichen am Kopfbereich halten. So kann der Griffbereich frei sein, um ungehindert von der Bedienperson gegriffen zu werden. Da der Kopfbereich des Scanners aber mit Speichel und Blut in Kontakt kommen kann, ist es bei dieser Art von Halterung erforderlich, den Teil der Halterung, welcher das Handstück hält regelmäßig zu desinfizieren. Da Halterungen für Handstücke von Intraoralscannern häufig im Bereich eines zahnärztlichen Behandlungsstuhles, direkt am Behandlungsstuhl oder am Scanner angebracht sind, lassen sich die Halterungen üblicherweise nur durch Abwischen desinfizieren. Dies ist in vielen Fällen unzureichend, um den strengen Hygieneanforderungen,, im medizinischen Bereich zu genügen. Vielmehr ist, insbesondere bei Instrumenten, welche mit dem Blut verschiedener Patienten in Berührung kommen können, üblicherweise ein Sterilisieren in einem Autoklaven erforderlich. Dies lässt sich bei Halterungen im Bereich des Behandlungsstuhles nur durch ein vollständiges Aus- bzw. Abbauen bewerkstelligen. Das ist zeitintensiv und aufwändig und daher impraktikabel. In Fällen, in denen die Halterung zusätzliche Aufgaben erfüllt, wie beispielsweise das Aufladen eines kabellosen Handstücks, kann es sogar nahezu unmöglich sein, die Halterung zur Reinigung zu entfernen.

Der Erfindung liegt daher die Aufgabe zu Grunde, die oben beschrieben Nachteile zu überwinden und eine Halterung für einen Intraoral-Scanner zu schaffen, die sowohl komfortabel als auch hygienisch und sicher ist.

Gelöst wird diese Aufgabe erfindungsgemäß durch eine Haltevorrichtung der eingangs genannten Art, die dadurch gekennzeichnet ist, dass die Halterung einen Einsatz aufweist, dass der Einsatz im Aufnahmebereich der Halterung wenigstens teilweise formschlüssig aufgenommen ist und dass der Kopfbereich des Handstücks wenigstens teilweise formschlüssig im Einsatz aufnehmbar ist.

Der Einsatz erfüllt dabei gleich mehrere Aufgaben. Zum einen trennt er den mit der Mundhöhle in Kontakt kommenden Kopfbereich des Scanners und die Halterung räumlich voneinander ab. So kann der Scanner die Halterung nicht beschmutzen. Umgekehrt kann die Halterung, selbst wenn diese nur durch Abwischen desinfiziert wurde, den Kopfbereich nicht kontaminieren. Zum anderen bildet der Einsatz einen Teil der Halterung, welcher ohne aufwändiges Ausbauen der übrigen Halterung nicht nur desinfiziert sondern, beispielsweise in einem Autoklaven, sterilisiert werden kann.

In einer bevorzugten Weiterbildung der Erfindung weist der Einsatz an einer Außenfläche wenigstens einen Vorsprung, vorzugsweise zwei Vorsprünge, auf der in eine Ausnehmung oder Vertiefung am Aufnahmebereich eingreift. So werden Rasten geschaffen welche die formschlüssige Verbindung zwischen Einsatz und Grundkörper bilden oder verstärken. Damit kann die Verbindung zwischen Grundkörper und Einsatz stärker als die formschlüssige Verbindung zwischen Scannerkopf und Einsatz gestaltet werden. Dadurch wird vermieden, dass sich der Einsatz aus dem Grundkörper löst, wenn der Scannerkopf aus der Halterung genommen wird. Zusätzlich oder alternativ kann der Grundkörper oder der Einsatz auch ein Mittel zum Verriegeln der Verbindung zwischen Grundkörper und Einsatz aufweisen. Dies kann beispielsweise ein verschiebbarer Riegel oder Bolzen sein.

In einer weiteren bevorzugten Ausführungsform der Erfindung weist der Einsatz eine Strahlengang-Ausnehmung auf, die mit einem Sichtfenster des Handstücks korrespondiert. Grundsätzlich benötigen alle Handstücke von optisch arbeitenden Intraoralscannern in ihrem Gehäuse einen freien Bereich, der es der im Handstück angeordneten Optik ermöglicht die zu erfassenden Objekte zu "sehen". Dieses Sichtfenster kann sowohl durch eine transparente Schutzschicht, beispielsweise durch ein Glas, abgedeckt als auch frei sein. Die Strahlengang-Ausnehmung im Einsatz kann dabei verschiedene Zwecke erfüllen. Zum einen kann, sofern im Sichtfenster des Handstücks eine transparente Schutzschicht vorgesehen ist, die Schutzschicht des Sichtfensters nicht durch Berührung mit dem Einsatz verschmutzt werden. Zum anderen können, wenn im Grundkörper Kalibriervorrichtungen oder -hilfen angeordnet sind, diese mit der Optik interagieren. Dazu ist, gemäß einer weiteren bevorzugten Ausführungsform der Erfindung, eine mit dem Strahlengang im Wesentlichen korrespondierende Kalibrier-Ausnehmung im Grundkörper angeordnet.

Gemäß bevorzugter Weiterbildungen der Erfindung können die Strahlengang-Ausnehmung und/oder die Kalibrier-Ausnehmung ebenfalls mit transparenten Schutzschichten wenigstens teilweise verschlossen sein. So kann ein Einsickern von flüssigen Verschmutzungen in den Grundkörper vermieden werden.

Bevorzugt besteht der Einsatz aus einem im Wesentlichen starren Material, insbesondere einem Kunststoff.

Weitere bevorzugte Ausführungsformen der Erfindung sind Gegenstand der übrigen Unteransprüche.

Nachstehend ist ein bevorzugtes Ausführungsbeispiel der Erfindung anhand der Zeichnungen näher beschrieben. Es zeigt:
- Fig. 1: eine erfindungsgemäße Halterung mit einem Grundkörper und einem Einsatz,
- Fig. 2: die Halterung von Fig. 1 mit einem Handstück eines Intraoralscanners,
- Fig. 3: das Handstück von Fig. 2 mit dem Einsatz,
- Fig. 4: den Einsatz,
- Fig. 5: einen schematisierten Längsschnitt durch Fig. 2,
- Fig. 6: einen schematisierten Querschnitt durch Fig. 2 und
- Fig. 7: einen schematisierten Längsschnitt durch eine Alternative Ausführungsform der Erfindung.

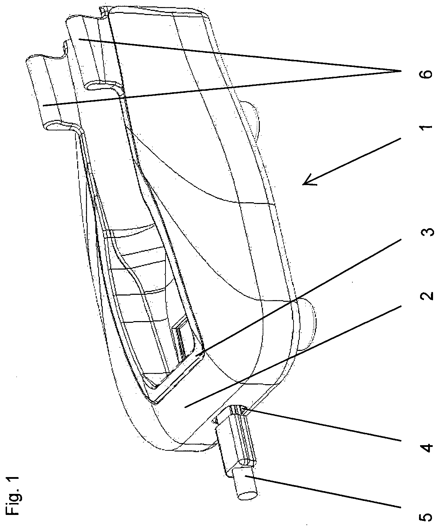

Fig. 1 zeigt eine erfindungsgemäße Halterung 1 mit einem Grundkörper 2 und einem formschlüssig im Grundkörper 1 aufgenommenen Einsatz 3. Am Grundkörper 3 befindet sich ein Anschluss 4 für ein Kabel 5. Dieses kann im Grundkörper 2 befindliche Elektronik (nicht dargestellt) mit einer Recheneinheit (nicht dargestellt) verbinden. Diese Elektronik kann verschiede Aufgaben erfüllen, wie beispielsweise ein Kalibrieren von in einem Handstück eines Intraoralscanners angeordneten optischen und/oder elektronischen Elementen, ein Vorheizen des Handstücks, um ein Beschlagen durch die Atemluft zu vermeiden, oder auch ein Kühlen, um ein Überhitzen des Handstücks, beispielsweise durch darin befindliche Leuchtmittel, zu vermeiden.

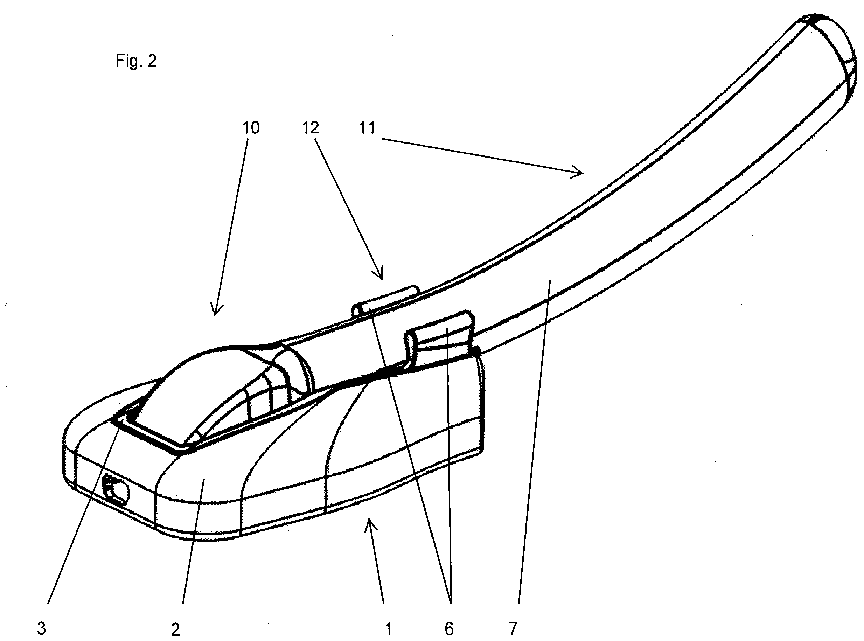

An einem Endbereich des Einsatzes 3 sind zwei Griffstücke 6 des Einsatzes 3 angeordnet. Diese dienen zum einen dazu, ein in der Halterung 1 gelagertes Handstück 7 eines Intraoralscanners (siehe Fig. 2) besser in der Halterung 1 zu halten. Zum anderen können die Griffstücke 6 verwendet werden, um den Einsatz 3 geringfügig elastisch zu verformen und so die formschlüssige Verbindung zwischen Einsatz 3 und Grundkörper 2 zu lösen.

In der dargestellten Ausführungsform ist die Halterung 1 so konstruiert, dass sie, beispielsweise auf einer Arbeitsfläche, frei stehen kann. Hierfür weist die Halterung 1 Füße 8 auf. Diese sind bevorzugt aus einem rutschfesten Material gefertigt. Es sind aber auch Ausführungsformen denkbar, bei denen die Halterung in einen Apparat oder Behandlungsstuhl integriert ist. Hierfür genügt eine Anpassung des Grundkörpers. Mögliche Varianten zur Ausgestaltung des Grundkörpers, die bei der vorliegenden Halterung verwendet werden können, können beispielsweise der AT 13546 U1 entnommen werden. Das Dokument AT 13546 U1 und die darin offenbarten Merkmale der Halterung werden daher hiermit in vollem Umfang durch Bezugnahme in dieses Dokument aufgenommen.

Fig. 2 zeigt die Halterung 1 mit einem Handstück 7 eines Intraoralscanners (nicht dargestellt) . Ein Kopfbereich 10 des Handstücks 7 ist dabei im Einsatz 3 formschlüssig aufgenommen, während ein Griffbereich 11 des Handstücks 7 für eine Bedienungsperson frei zugänglich und bequem zu ergreifen ist. Die Griffstücke 6 des Einsatzes 3 halten das Handstück 7 knapp hinter dem Kopfbereich 10 in einem Zwischenbereich 12.

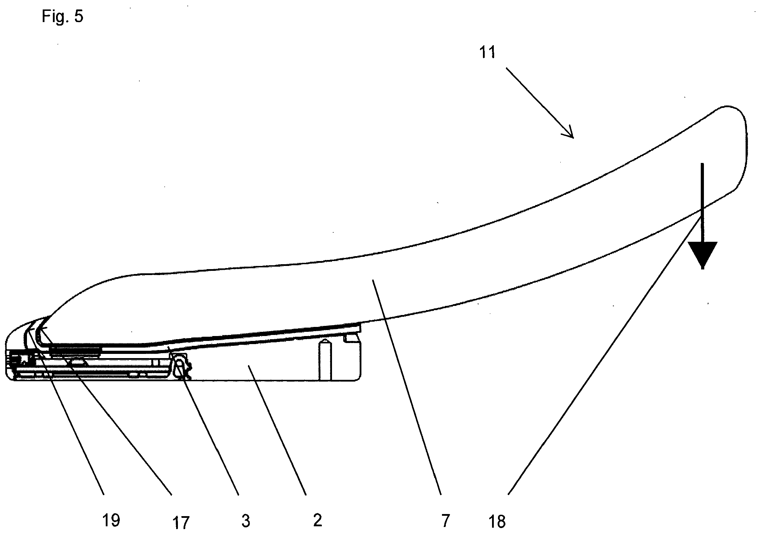

Die Anordnung der Griffstücke 6 hat dabei verschiedene Vorteile. Da die Griffstücke 6 bei einem Einsetzen des Einsatzes 3 in den Grundkörper 2 angefasst werden, ist es für die Hygiene bei der Verwendung der Halterung 1 förderlich, wenn die Griffstücke 6 so angeordnet sind, dass sie nicht mit dem Kopfbereich 10 in Berührung kommen, gleichzeitig aber den Griff bereich 11 frei lassen. Die übrigen Vorteile, die sich aus der Anordnung der Griff stücke 6 ergeben, sind mechanischer Natur und werden in Verbindung mit der Fig. 5 beschrieben, auf welche später näher eingegangen wird.

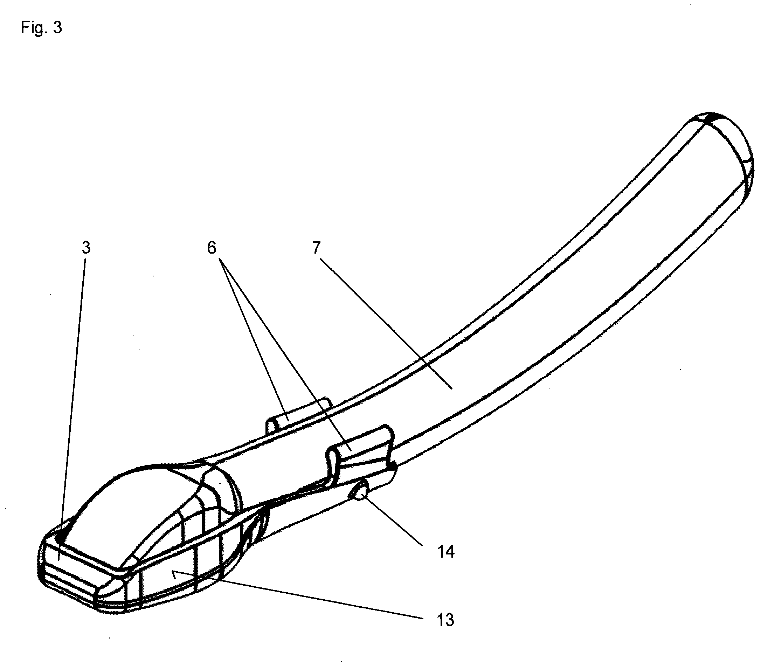

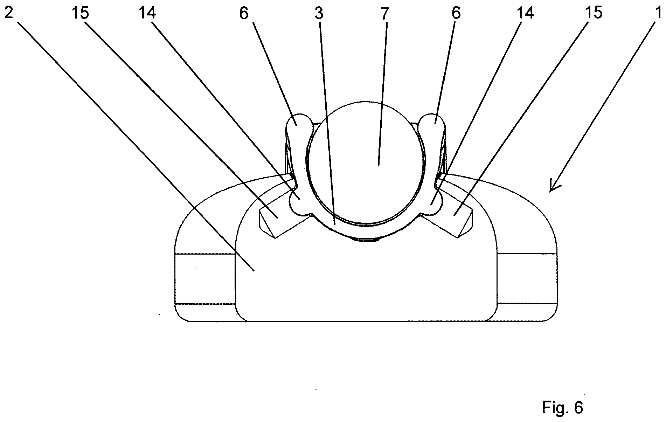

Fig. 3 zeigt das Handstück 7 zusammen mit dem Einsatz 3. An einer Außenseite 13 des Einsatzes 3 erkennt man unterhalb der Griffstücke 6 einen von zwei auf gegenüberliegenden Seiten des Einsatzes 3 angeordneten Vorsprüngen 14. Diese können in korrespondierende Ausnehmungen oder Vertiefungen 15 (siehe Fig. 6) im Grundkörper 2 eingreifen und so die Verbindung zwischen Grundkörper 2 und Einsatz 3 verstärken. Selbstverständlich können die Vorsprünge 14 auch an einem anderen Bereich des Einsatzes 3 angeordnet sein, allerdings werden die Vorsprünge 14, wenn diese im Bereich der Griff stücke 6 angeordnet sind, mit den Griffstücken 6 gemeinsam bewegt, wenn die Griffstücke 6 verformt werden. So kann der Einsatz auch bei einer durch die Vorsprünge 14 verstärkten formschlüssigen Verbindung mit dem Grundkörper 2 bequem von diesem entfernt werden.

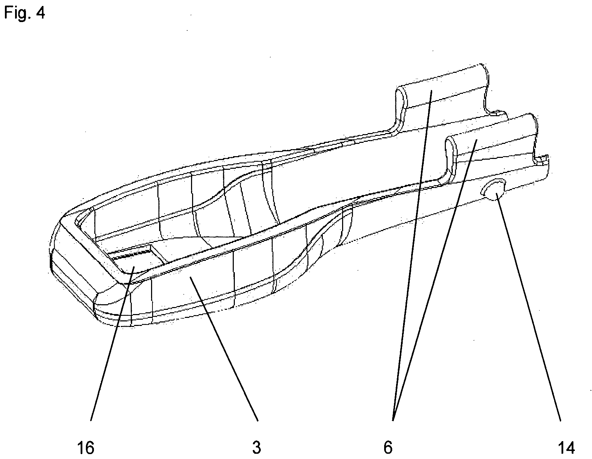

Fig. 4 zeigt den Einsatz 3 ohne Grundkörper 2 und Handstück 7. Man erkennt eine Strahlengang-Ausnehmung 16 in einem unteren Bereich des Einsatzes 3. Diese Strahlengang-Ausnehmung 16 ist, wenn ein Handstück 7 in der Halterung 1 abgelegt ist, einem Sichtfenster (nicht dargestellt) des Handstücks zugeordnet angeordnet. Die Strahlengang-Ausnehmung 16 kann dabei helfen, ein Abdeckglas des Sichtfensters weniger zu verschmutzen oder um einen optischen oder elektromagnetischen Weg zwischen dem Handstück 7 bzw. darin angeordneten elektronischen und/oder optischen Komponenten und elektronischen und/oder optischen Elementen im Grundkörper zu ermöglichen oder zu erleichtern.

Fig. 5 zeigt einen Schnitt durch die in Fig. 2 gezeigte Halterung 1 mit dem darin angeordneten Handstück 7. Der Übersicht halber sind mögliche elektronisch und/oder optische Komponenten im Handstück 7 nicht dargestellt. Weiters erkennt man gegenüber dem Kopfbereich 10 des Handstücks 7 eine von oben am Scannerkopf angreifende Einsatz-Haltefläche 17. Wirkt auf den Griffbereich 11 des Handstücks 7 eine nach unten wirkende Kraft (dargestellt durch einen Pfeil 18), beispielsweise die Schwerkraft, wirkt die Einsatz-Haltefläche 17 dieser entgegen und der Scanner wird in der Halterung 1, respektive dem Einsatz 3 gehalten. Wird die Halterung 1 nicht in einer im Wesentlichen horizontalen Ausrichtung angeordnet, können die Griffstücke 6 (vgl. Fig. 2) stattdessen auf das Handstück 7 wirken und so für Halt sorgen. Selbstverständlich wirken die Griff stücke 6 auch in einer im Wesentlichen horizontalen Anordnung der Halterung 1 unterstützend für einen Halt des Handstücks 7 in der Halterung 1.

Analog zur Einsatz-Haltefläche 17 weist der Grundkörper 2 einen Grundkörper-Haltebereich 19 auf, welcher von vorne und oben am Einsatz 3 angreift. Die Vorsprünge 14 (Fig. 2 und Fig. 3) sorgen dafür, dass der Einsatz in dieser Stellung gehalten wird. Es sind aber auch andere Varianten denkbar, bei denen beispielsweise eine lösbare Verriegelung den Einsatz 3 gegenüber dem Grundkörper 2 fixiert.

Fig. 6 zeigt einen weiteren Schnitt durch die in Fig. 2 gezeigte Halterung. Darin erkennt man, wie die Vorsprünge 14 in die dafür vorgesehenen Vertiefungen 15 eingreifen. Statt Vertiefungen 15 kann der Grundkörper 2 an dieser Stelle natürlich auch einfach nur Ausnehmungen aufweisen.

Man erkennt, dass eine Bewegung des Handstücks 7, wenn dieses aus der Halterung 1 entfernt wird, die Griffstücke 6 nach außen drückt. Dadurch werden die Rasten 14 stärker in die Vertiefungen 15 gedrückt. Die günstige Anordnung der Griffstücke 6 und der Rasten 14 zueinander verhindert so, dass der Einsatz 3 mit dem Handstück 7 aus dem Grundkörper 2 gehoben wird.

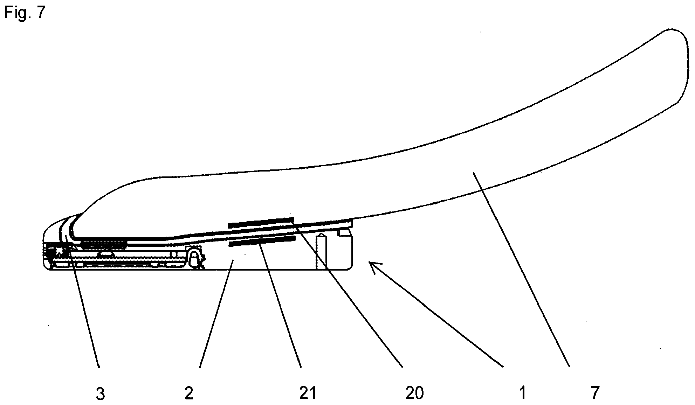

Fig. 7 zeigt einen Schnitt durch eine alternative Ausführungsform einer erfindungsgemäßen Halterung. Bei dieser wird die auf das Handstück wirkende Kraft, welche den Kopfbereich gegen den Einsatz-Haltebereich 13 drückt, mittels magnetischer Elemente 20, 21 unterstütz bzw. je nach Lagerung der Halterung 1 bewirkt. Bei den magnetischen Elementen kann es sich beispielsweise um einen Magnet und ein Metallplättchen oder zwei Magnete handeln. Die Wahl, welche Paarung dabei sinnvoll ist und ob bei einer Metall-Magnet-Paarung der Magnet im Handstück 7 oder im Grundkörper 2 angeordnet wird, hängt von verschiedenen Faktoren ab, wie beispielsweise der Anordnung von möglicherweise im Handstück 7 und/oder im Grundkörper 2 vorgesehener Elektronik, und kann vom Fachmann entsprechend gewählt werden.

## Patentansprüche

1. Halterung (1) in Verbindung mit einem Handstück (7) eines Intraoralscanners, welches einen Kopfbereich (10) aufweist, und mit einem Grundkörper (2) mit einem Aufnahmebereich, in welchem der Kopfbereich aufnehmbar ist, **dadurch gekennzeichnet, dass** die Halterung einen Einsatz (3) aufweist, dass der Einsatz im Aufnahmebereich der Halterung wenigstens teilweise formschlüssig aufgenommen ist und dass der Kopfbereich des Handstücks wenigstens teilweise formschlüssig im Einsatz aufnehmbar ist.

2. Halterung nach Anspruch 1, **dadurch gekennzeichnet, dass** das der Einsatz an einer Außenfläche wenigstens einen Vorsprung (14), vorzugsweise zwei Vorsprünge, aufweist, der in eine Ausnehmung oder Vertiefung (15) am Aufnahmebereich eingreift.

3. Halterung nach Anspruch 2, **dadurch gekennzeichnet, dass** der Vorsprung im Wesentlichen kugelsegmentförmig ausgebildet ist.

4. Halterung nach einem der Ansprüche 1 bis 3, **dadurch gekennzeichnet, dass** der Einsatz eine Strahlengang-Ausnehmung (16) aufweist, die mit einem Sichtfenster des Handstücks im Wesentlichen korrespondiert.

5. Halterung nach Anspruch 4, **dadurch gekennzeichnet, dass** die Strahlengang-Ausnehmung mit einem transparenten Material verschlossen ist.

6. Halterung nach Anspruch 4 oder 5, **dadurch gekennzeichnet, dass** die Strahlengang-Ausnehmung mit einer Kalibrier-Ausnehmung des Grundkörpers im Wesentlichen korrespondiert.

7. Halterung nach einem der Ansprüche 1 bis 6, **dadurch gekennzeichnet, dass** der Einsatz wenigstens oberflächlich aus einem biokompatiblen Material besteht.

8. Halterung nach einem der Ansprüche 1 bis 7, **dadurch gekennzeichnet, dass** der Grundkörper wenigstens eine Metallplatte und/oder einen Magnet (20, 21) aufweist, der mit einem Magnet und/oder einer Metallplatte im Handstück korrespondiert.

9. Halterung nach einem der Ansprüche 2 bis 8, **dadurch gekennzeichnet, dass** der Einsatz Griffstücke (6) aufweist.

10. Halterung nach Anspruch 9, **dadurch gekennzeichnet, dass** die Griff stücke den Scanner halten.

11. Halterung nach Anspruch 9 oder 10, **dadurch gekennzeichnet, dass** die Griffstücke im Bereich der Vorsprünge (14) angeordnet sind.

## Claims

1. Holder (1) in conjunction with a hand piece (7) of an intraoral scanner having a head region (10) and with a main body (2) having a receiving region in which the head region can be accommodated, **characterized in that** the holder is provided with an insert (3), **in that** in the receiving region of the holder and at least portions of the insert are interlocked with each other and **in that** at least portions of the head region of the hand piece can be interlocked in the insert.

2. Holder as claimed in claim 1, **characterized in that** an outer surface of the insert is provided with at least one protrusion (14), preferably two protrusions which engage in a recess or depression (15) in the receiving region.

3. Holder as claimed in claim 2, **characterized in that** the protrusion is essentially in the shape of a spherical segment.

4. Holder as claimed in one of claims 1 to 3, **characterized in that** the insert is provided with a beam path clearance (16) which essentially corresponds with a window of the hand piece.

5. Holder as claimed in claim 4, **characterized in that** the beam path clearance is closed with a transparent material.

6. Holder as claimed in claim 4 or claim 5, **characterized in that** the beam path clearance essentially corresponds with a calibration clearance of the main body.

7. Holder as claimed in one of claims 1 to 6, **characterized in that** at least the surface of the insert consists of a biocompatible material.

8. Holder as claimed in one of claims 1 to 7, **characterized in that** the main body is provided with at least one metal plate and/or a magnet (20, 21) which corresponds with a magnet and/or a metal plate in the hand piece.

9. Holder as claimed in one of claims 2 to 8, **characterized in that** the insert is provided with handle pieces (6).

10. Holder as claimed in claim 9, **characterized in that** the handle pieces retain the scanner.

11. Holder as claimed in claim 9 or claim 10, **characterized in that** the handle pieces are disposed in the region of the protrusions (14).

## Revendications

1. Fixation (1) en association avec une pièce à main (7) d'un scanner intra-oral, laquelle comporte une zone de tête (10), et avec un corps de base (2) avec une zone de logement dans laquelle la zone de tête peut être réceptionnée, **caractérisée en ce que** la fixation comporte un insert (3) **en ce que** l'insert est réceptionné au moins en partie par complémentarité de forme dans la zone de logement de la fixation et **en ce que** la zone de tête de la pièce à main peut être réceptionné au moins en partie par complémentarité de forme dans l'insert.

2. Fixation selon la revendication 1, **caractérisée en ce que** sur une surface extérieure, l'insert comporte au moins une saillie (14), de préférence deux saillies qui s'engage(nt) dans un évidement ou dans un creux (15) sur la zone de logement.

3. Fixation selon la revendication 2, **caractérisée en ce que** la saillie est sensiblement conçue en forme de segment sphérique.

4. Fixation selon l'une quelconque des revendications 1 à 3, **caractérisée en ce que** l'insert comporte un exclusion (16) de trajectoire de rayons qui correspond sensiblement avec une fenêtre de la pièce à main.

5. Fixation selon la revendication 4, **caractérisée en ce que** l'exclusion de trajectoire des rayons est fermé par une matière transparente.

6. Fixation selon la revendication 4 ou la revendication 5, **caractérisée en ce que** l'exclusion de trajectoire des rayons correspond sensiblement avec un exclusion de calibrage du corps de base.

7. Fixation selon l'une quelconque des revendications 1 à 6, **caractérisée en ce que** l'insert est composé au moins en surface d'une matière biocompatible.

8. Fixation selon l'une quelconque des revendications 1 à 7, **caractérisée en ce que** le corps de base comporte au moins une plaque métallique et/ou un aimant (20, 21) qui correspond avec un aimant et/ou avec une plaque métallique dans la pièce à main.

9. Fixation selon l'une quelconque des revendications 2 à 8, **caractérisée en ce que** l'insert comporte des poignées (6).

10. Fixation selon la revendication 9, **caractérisée en ce que** les poignées tiennent le scanner.

11. Fixation selon la revendication 9 ou la revendication 10, **caractérisée en ce que** les poignées sont placées dans la zone des saillies (14).
